# EUROPÄISCHE PATENTSCHRIFT

(11) **EP 4 522 063 B1**
(45) Veröffentlichungstag und Bekanntmachung des Hinweises auf die Patenterteilung: **01.10.2025**
(21) Anmeldenummer: 23726075.7
(22) Anmeldetag: 10.05.2023
(51) Int. Cl.: A61B 50/13, A61B 50/18, A61B 50/10

(54) **TRANSPORTVORRICHTUNG ZUM TRANSPORT ZUMINDEST EINES STERILGUTBEHÄLTERS**
TRANSPORT DEVICE FOR TRANSPORTING AT LEAST ONE STERILE-PRODUCT CONTAINER
DISPOSITIF DE TRANSPORT POUR LE TRANSPORT D'AU MOINS UN RÉCIPIENT DE PRODUIT STÉRILE

(30) Priorität: 10.05.2022 DE 102022111697
(43) Veröffentlichungstag der Anmeldung: 19.03.2025
(73) Patentinhaber: Aesculap AG, 78532 Tuttlingen (DE)
(72) Erfinder: JANSEN-TROY, Arne, 78333 Stockach (DE); MAAS, Allan, 78467 Konstanz (DE); GOERZ, Dennis, 78532 Tuttlingen (DE); DORAU, Stephan, 91074 Herzogenaurach (DE); ELISCH, Andreas, 78655 Dunningen (DE); THOMAS, Stefan, 78532 Tuttlingen (DE); SCHWEIZER, Matthias, 78532 Tuttlingen (DE); KIESSLING, Daniel, 78052 Villingen-Schwenningen (DE); HENKE, Matthias, 78048 Villingen-Schwenningen (DE)
(74) Vertreter: Winter, Brandl - Partnerschaft mbB
(86) Internationale Anmeldenummer: PCT/EP2023/062498
(87) Internationale Veröffentlichungsnummer: WO 2023/217905

(56) Entgegenhaltungen:
- EP-B1- 2 594 453
- WO-A1-2014/092331
- WO-A1-2021/068716
- CN-A- 110 680 507
- CN-A- 113 133 834
- CN-A- 113 442 979
- DE-A1- 102016 121 749

## Beschreibung

### Technisches Gebiet

Die vorliegende Erfindung bezieht sich auf eine Transportvorrichtung zum Transport zumindest eines Sterilgutbehälters mit einem Aufnahmeraum, der auf einer ersten Ebene und auf einer zu der ersten Ebene parallelen zweiten Ebene Auflageelemente aufweist, auf denen eine, insbesondere als Tablar ausgebildete, Sterilgutbehälteraufnahme lagerbar ist, und einer Hebeauflage, die in einer ersten Position derart an die Auflageelemente der ersten Ebene anordenbar ist, dass die Sterilgutbehälteraufnahme zwischen den Auflageelementen der ersten Ebene und der Hebeauflage hin und her verschiebbar ist.

### Stand der Technik

EP 2 594 453 B1 offenbart eine Transportvorrichtung zum Transport zumindest eines Sterilgutbehälters mit einem Aufnahmeraum, der auf einer ersten Ebene und auf einer zu der ersten Ebene parallelen zweiten Ebene Auflageelemente aufweist, auf denen eine Sterilgutbehälteraufnahme lagerbar ist. Die bekannte Transportvorrichtung weist eine Hebeauflage bzw. eine Hubeinrichtung auf, die derart an die Auflageelemente der ersten Ebene anordenbar ist, dass die Sterilgutbehälteraufnahme zwischen den Auflageelementen der ersten Ebene und der Hebeauflage hin und her verschiebbar ist.

Eine gattungsgemäße Transportvorrichtung wird in DE 10 2016 121 749 A1 offenbart.

Problem der Transportvorrichtung gemäß EP 2 594 453 B1 ist, dass deren Hebeauflage bzw. Hubeinrichtung nur für Auflageelemente einer einzigen Ebene ausgelegt ist. Die bekannte Hubeinrichtung dient lediglich dazu, eine kleine Sterilgutbehälteraufnahme in Form eines Einschubgestells in einen Transportwagen einsetzen zu können, welcher eigentlich für den Transport einer größeren Sterilgutbehälteraufnahme in Form eines Einschubgestells ausgebildet ist.

### Zusammenfassung der Offenbarung

Aufgabe der vorliegenden Offenbarung ist es deshalb, eine Transportvorrichtung zum Transport zumindest eines Sterilgutbehälters bereitzustellen, die einen platzsparenden Transport mehrerer Sterilgutbehälter in ergonomischer Weise ermöglicht.

Diese Aufgabe wird durch eine Transportvorrichtung mit den Merkmalen des Anspruchs 1 bzw. deren Verwendung zum Transport zumindest eines Sterilgutbehälters gelöst. Vorteilhafte Weiterbildungen sind Gegenstand der Unteransprüche.

Eine offenbarungsgemäße Transportvorrichtung zum Transport zumindest eines Sterilgutbehälters weist einen Aufnahmeraum und eine Hebeauflage auf.

Der Aufnahmeraum ist in einem beweglichen Transportwagen ausgebildet und weist auf einer ersten Ebene und auf einer zu der ersten Ebene parallelen zweiten Ebene Auflageelemente auf, auf denen eine Sterilgutbehälteraufnahme lagerbar ist. Die Auflageelemente können insbesondere an Seitenwänden des Aufnahmeraums hervorstehende Vorsprünge sein, die nach oben gewandte Flächen bzw. Kontaktflächen aufweisen, welche mit der Sterilgutbehälteraufnahme bzw. mit nach unten gewandten Flächen bzw. Kontaktflächen der Sterilgutbehälteraufnahme, insbesondere flächig, in Kontakt treten können. Die Sterilgutbehälteraufnahme kann insbesondere als ein Tablar ausgebildet sein. Alternativ kann die Sterilgutbehälteraufnahme auch als ein Einschubgestell ausgebildet sein.

Die Hebeauflage ist ausgebildet, in einer ersten Position derart an die Auflageelemente der ersten Ebene angeordnet zu werden bzw. werden zu können, dass die Sterilgutbehälteraufnahme zwischen den Auflageelementen der ersten Ebene und der Hebeauflage hin und her verschiebbar ist. Insbesondere kann die Hebeauflage ausgebildet sein, in der ersten Position derart an die Auflageelemente der ersten Ebene angeordnet zu werden bzw. werden zu können, dass sich die Hebeauflage parallel zu den Auflageelementen der ersten Ebene bzw. den Kontaktflächen der Auflageelemente der ersten Ebene erstreckt und insbesondere mit diesen fluchten.

Des Weiteren ist die Hebeauflage ausgebildet, in einer zweiten Position derart an die Auflageelemente der zweiten Ebene angeordnet zu werden bzw. werden zu können, dass die Sterilgutbehälteraufnahme zwischen den Auflageelementen der zweiten Ebene und der Hebeauflage hin und her verschiebbar ist. Insbesondere kann die Hebeauflage ausgebildet sein, in der zweiten Position derart an die Auflageelemente der zweiten Ebene angeordnet zu werden bzw. werden zu können, dass sich die Hebeauflage parallel zu den Auflageelementen der zweiten Ebene bzw. den Kontaktflächen der Auflageelemente der zweiten Ebene erstreckt und insbesondere mit diesen fluchten.

Die Transportvorrichtung weist eine Linearführung, insbesondere in Form zumindest einer Führungsschiene, auf, die quer, insbesondere senkrecht, zu der ersten und zweiten Ebene verläuft und mittels derer die Hebeauflage von deren ersten Position in deren zweite Position geführt werden kann. Außerdem weist die Transportvorrichtung einen, insbesondere in der Linearführung bzw. der Führungsschiene integrierten, Antrieb, insbesondere einen Linearantrieb, vorzugsweise in Form eines elektromechanischen Aktors, auf, mittels dessen die Hebeauflage entlang der Linearführung von der ersten Position in die zweite Position bewegbar ist.

Die Linearführung, der Antrieb und die Hebeauflage sind an dem Transportwagen ausgebildet.

Die offenbarungsgemäße Transportvorrichtung ermöglicht es in vorteilhafterweise, den Aufnahmeraum, ohne eine übermäßige körperliche Belastung umfassender mit Sterilgutbehältern zu befüllen.

Gemäß einem Aspekt der Offenbarung kann der Transportwagen einen Rahmen aufweisen, der eine Öffnung einfasst, durch welche der Sterilgutbehälter in den oder aus dem Aufnahmeraum bewegt werden kann, kann der Transportwagen eine Tür aufweisen, die schwenkbar an einer äußeren Stirnseite des Rahmens angebracht ist, und können die Linearführung, der Antrieb sowie die Hebeauflage an einer Innenseite der Tür derart ausgebildet sein, dass die Hebeauflage in einer geöffneten Stellung der Tür parallel zu den Auflageelementen des Aufnahmeraums ausgerichtet sein kann.

Wird die Hebeauflage separat von der Tür an der Innenseite der Tür ausgebildet, kann die Hebeauflage in vorteilhafter Weise vor äußeren Einflüssen geschützt werden.

Gemäß einem Aspekt der Offenbarung kann die Hebeauflage derart schwenkbar mit der Linearführung verbunden sein, dass die Hebeauflage aus einer ausgeklappten Stellung, in welcher die Hebeauflage in der geöffneten Stellung der Tür parallel zu den Auflageelementen des Aufnahmeraums ausgerichtet ist, in eine eingeklappte Stellung geklappt werden kann, in welcher die Hebeauflage bzw. deren Kontaktfläche, die für den Kontakt mit der Sterilgutbehälteraufnahme ausgelegt bzw. ausgebildet ist, parallel zu der Tür bzw. deren Innenseite ausgerichtet ist.

Wird die Hebeauflage schwenkbar mit der Linearführung ausgebildet, können die Ausmaße der Hebeauflage in vorteilhafterweise an die Größe der Sterilgutbehälteraufnahme bzw. des zu transportierenden Sterilgutbehälters angepasst werden.

Gemäß einem Aspekt der Offenbarung kann die Tür als Stulptür mit einem ersten Türblatt und einem zweiten Türblatt ausgebildet sein, und kann die Hebeauflage ein erstes Hebeauflageelement aufweisen, das an dem ersten Türblatt ausgebildet ist, sowie ein zweites Hebeauflagenelement aufweisen, das an dem zweiten Türblatt ausgebildet ist. Ein Stulp wird insbesondere durch einen mittigen Abschnitt des Rahmens gebildet. Anders ausgedrückt kann die Tür als eine doppelflügelige Schwenktür ausgebildet sein, deren beide Türflügel derart schwenkbar mit dem Rahmen verbunden sind, dass, insbesondere senkrecht zu der ersten Ebene verlaufende, Schwenkachsen der beiden Türflügel an zwei gegenüberliegenden Seiten des Rahmens angeordnet sind.

Wird die Tür als Stulptür bzw. doppelflügelige Schwenktür ausgebildet, kann eine bei einem Verschieben des Sterilgutbehälters zwischen der ersten und zweiten Ebene auftretende Last besser verteilt werden.

Gemäß einem Aspekt der Offenbarung kann der Transportwagen einen Rahmen aufweisen, der eine Öffnung einfasst, durch welche der Sterilgutbehälter in den oder aus dem Aufnahmeraum bewegt werden kann, und können die Linearführung, der Antrieb sowie die Hebeauflage an einer äußeren Stirnseite des Rahmens ausgebildet sein.

Werden die Linearführung, der Antrieb und die Hebeauflage an bzw. integral mit dem Transportwagen ausgebildet, kann eine ergonomische Be- und Entladbarkeit ortsunabhängig ermöglicht werden.

Gemäß einem Aspekt der Offenbarung kann die Hebeauflage als eine Tür ausgebildet sein, die derart schwenk- und verschiebbar mit der Linearführung verbunden ist, dass die Tür bzw. Hebeauflage in einer Abdeckungsorientierung die von dem Rahmen eingefasste Öffnung zumindest größtenteils, insbesondere vollständig, abdecken kann und die Tür bzw. Hebeauflage in einer quer, insbesondere senkrecht, zur Abdeckungsorientierung ausgerichteten Hebeorientierung, in welcher insbesondere die Öffnung teilweise, insbesondere größtenteils, nicht durch die Tür bzw. Hebeauflage abgedeckt ist, parallel zu den Auflageelementen bzw. dessen Kontaktflächen des Aufnahmeraums ausgerichtet ist.

Wird die Hebeauflage als Tür ausgebildet, kann die ergonomische Be- und Entladbarkeit des Transportwagens auf effiziente Art realisiert werden.

Gemäß einem Aspekt der Offenbarung kann die Tür als eine Falttür ausgebildet sein, deren Türblätter in einem auseinandergefalteten Zustand in der Abdeckungsorientierung und in einem zusammengefalteten Zustand in der Hebeorientierung ausgerichtet sind. Insbesondere kann die Linearführung zwei Führungsschienen aufweisen, die an gegenüberliegenden Seiten der Stirnseite des Rahmens angeordnet sind. Insbesondere kann der Antrieb je Führungsschiene einen Aktor zum Verschieben der Türblätter aufweisen.

Wird die Hebeauflage als Falttür ausgebildet, können die Ausmaße der Hebeauflage in vorteilhafterweise auf die Größe der Sterilgutbehälteraufnahme bzw. des zu transportierenden Sterilgutbehälters angepasst werden.

Gemäß einem Aspekt der Offenbarung kann der Antrieb je Türblatt zumindest einen Aktor zum Verschieben des jeweiligen Türblatts entlang der Linearführung aufweisen. Insbesondere kann die Linearführung zwei Führungsschienen aufweisen, die an gegenüberliegenden Seiten der Stirnseite des Rahmens angeordnet sind. Insbesondere kann der Antrieb je Türblatt und je Führungsschiene einen Aktor zum Verschieben des jeweiligen Türblatts aufweisen. Insbesondere können die Aktoren der einen der beiden Führungsschienen synchron mit den Aktoren der anderen der beiden Führungsschienen betätigt werden.

Wird je Türblatt zumindest ein Aktor vorgesehen, ist es in vorteilhafter Weise möglich, ein Verschieben der Hebeauflage relativ zu der ersten und zweiten Ebene und auch ein Ent- und/oder Zusammenfalten der Falttür automatisch durchführen zu können.

Gemäß einem Aspekt der Offenbarung kann an der Tür zumindest ein relativ zur Tür bewegliches Abstützelement vorgesehen sein, das in eine Abstützposition, insbesondere linear, bewegbar ist, in welcher das Abstützelement die Tür in deren geöffneten Stellung gegenüber einem planen Untergrund, auf dem der Transportwagen lagert, abstützen kann. Insbesondere kann der Transportwagen je Türflügel zumindest ein Abstützelement aufweisen. Vorzugsweise kann je freiem Ende jedes Türflügels zumindest ein Abstützelement an dem jeweiligen freien Ende vorgesehen sein.

Weist der Transportwagen zumindest ein Abstützelement auf, kann eine Wahrscheinlichkeit für ein Umstürzen des Transportwagens bei geöffneter Tür in vorteilhafter Weise verringert werden.

Die vorliegende Offenbarung betrifft des Weiteren eine Verwendung einer offenbarungsgemäßen Transportvorrichtung zum Transport zumindest eines Sterilgutbehälters.

### Kurzbeschreibung der Zeichnungen

Die vorliegende Erfindung wird im Folgenden anhand bevorzugter Ausführungsbeispiele unter Bezugnahme auf die beigefügten Zeichnungen näher beschrieben. Es zeigen:
Figuren 1 bis 4 perspektivische Ansichten einer nicht von der vorliegenden Erfindung umfassten Ausführungsform einer Transportvorrichtung in verschiedenen Stellungen einer Hebeauflage bzw. einer Sterilgutbehälteraufnahme;
Figuren 5 bis 14 perspektivische Ansichten einer ersten Ausführungsform einer offenbarungsgemäßen Transportvorrichtung in verschiedenen Stellungen einer Hebeauflage bzw. einer Sterilgutbehälteraufnahme; und
Figuren 15 bis 22 perspektivische Ansichten einer zweiten Ausführungsform einer offenbarungsgemäßen Transportvorrichtung in verschiedenen Stellungen einer Hebeauflage bzw. einer Sterilgutbehälteraufnahme;

### Detaillierte Beschreibung bevorzugter Ausführungsformen

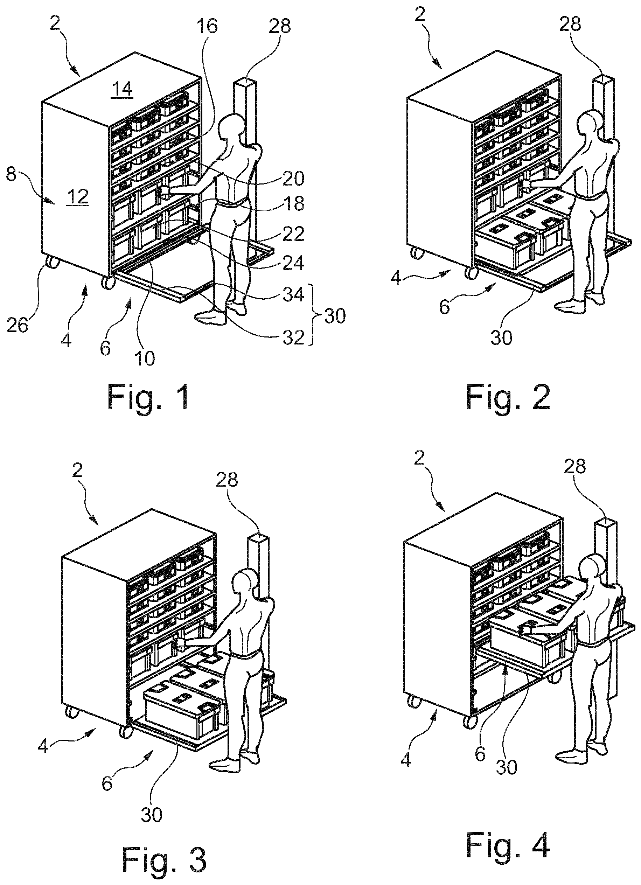

Fig. 1 zeigt eine perspektivische Ansicht einer nicht von der vorliegenden Erfindung umfassten Transportvorrichtung 2 mit einem Transportwagen 4 und einer von dem Transportwagen separaten, stationären Hebevorrichtung 6.

Der Transportwagen 2 ist als ein Rollschrank ausgebildet und weist ein Gehäuse 8 mit einem Boden 10, zwei sich von dem Boden 10 nach oben erstreckenden, sich gegenüberliegende Seitenwänden 12 und einer dem Boden 10 gegenüberliegenden Abdeckplatte 14 auf. Das Gehäuse 8 bzw. der Boden 10, die Seitenwände 12 und die Abdeckplatte 14 umrahmen einen Aufnahmeraum 16. An Innenseiten bzw. an zu dem Aufnahmeraum 16 zugewandte Seiten der Seitenwände 12 sind Auflageelemente 18 ausgebildet, die in den Aufnahmeraum 16 hineinragen. Die Auflageelemente 18 sind vorzugsweise als Leisten ausgebildet. Die Auflageelemente 18 sind derart angeordnet, dass zwei gegenüberliegende Auflageelemente 18 jeweils auf einer gemeinsamen Ebene liegen.

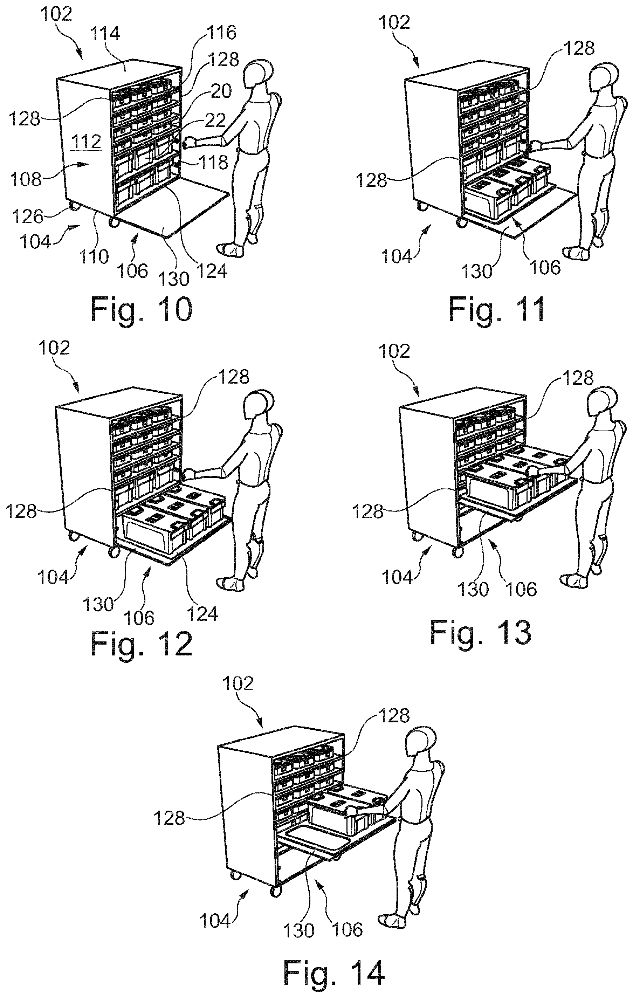

Um Sterilgutbehälter 20 bzw. 22 aufnehmen bzw. lagern zu können, weist der Transportwagen 4 Sterilgutbehälteraufnahmen 24 in Form von Tablaren auf. Die Sterilgutbehälteraufnahmen 24 sind derart dimensioniert, dass sie jeweils auf zwei sich gegenüberliegenden Auflageelementen 18 aufgelegt werden können und sich in diesem Zustand insbesondere parallel zu dem Boden 10 erstrecken. Die Sterilgutbehälteraufnahmen 24 weisen Geometrien wie Vertiefungen und/oder Erhebungen auf (siehe Fig. 14), um ein definiertes Platzieren der Sterilgutbehälter 20 bzw. 22 zu gewährleisten und ein Verrutschen der Sterilgutbehälter 20 bzw. 22 während eines Transports zu verhindern bzw. zumindest zu erschweren.

Die Sterilgutbehälteraufnahmen 24 können derart voneinander beabstandet in den Transportwagen 2 bzw. auf Auflageelemente 18 eingelegt werden, dass zwischen einzelnen (vorzugsweise unteren) Sterilgutbehälteraufnahmen 24 bzw. zwischen einer der Sterilgutbehälteraufnahmen 24 und dem Boden 10 / der Abdeckplatte 14 genug Platz für große Sterilgutbehälter 22 ist und dass zwischen anderen Sterilgutbehälteraufnahmen 24 bzw. zwischen einer der Sterilgutbehälteraufnahmen 24 und dem Boden 10 / der Abdeckplatte 14 nur genug Platz für kleine Sterilgutbehälter 20 ist.

Eine obere Seite des Bodens 10 ist insbesondere derart ausgebildet, dass diese ebenfalls ein Auflageelement 18 bildet bzw. Auflageelemente 18 aufweist.

Die Auflageelemente 18 können mit (nicht gezeigten) Geometrien ausgebildet sein, um ein definiertes Platzieren der Sterilgutbehälteraufnahmen 24 zu gewährleisten und ein Verrutschen der Sterilgutbehälteraufnahmen 24 während eines Transports zu verhindern bzw. zumindest zu erschweren. Diese Geometrien an den Auflageelementen 18 können insbesondere beweglich ausgebildet sein, um in einer Sicherungsstellung eine Sterilgutbehälteraufnahme 24 sichern und in einer Offenstellung ein Verschieben der Sterilgutbehälteraufnahme 24 zulassen zu können.

Um ein Bewegen des Transportwagens 4 zu ermöglichen, sind an einer unteren Seite des Bodens 10, jeweils in der Nähe einer Ecke des Bodens 10, Rollen 26 vorgesehen, die insbesondere als Lenkrollen ausgebildet sind.

Die Hebevorrichtung 6 weist eine Linearführung 28 mit einem darin integrierten (nicht gezeigten) Antrieb, insbesondere in Form eines Linearantriebs, und eine mit der Linearführung 28 verschiebbar verbundene Hebeauflage 30 auf.

Die Linearführung 26 ist fest mit einem Untergrund verbunden und erstreckt sich im Wesentlichen senkrecht.

Die Hebeauflage 30 weist zwei sich gegenüberliegenden Hebeauflageelemente 32 auf, die miteinander über Verbindungsstege 34 verbunden sind. Die Hebeauflageelemente 32 und die Verbindungstege 34 bilden zusammen einen rechteckigen Rahmen. Die Hebeauflageelemente 32 sind an einer Oberseite der Hebeauflage 30 gegenüber den Verbindungsstegen 34 derart überhöht, dass die Hebeauflageelemente 32 Schienen für die Sterilgutbehälteraufnahmen 24 bilden können.

In dem in Figur 1 gezeigten Zustand befindet sich die Hebeauflage 30 in einer untersten Position bzw. Stellung, in welcher die Hebeauflage 30 derart an die obere Seite des Bodens 10 bzw. an die Auflageelemente 18 des Bodens 10 angeordnet ist, dass die entsprechende unterste Sterilgutbehälteraufnahme 24 zwischen der oberen Seite des Bodens 10 bzw. den Auflageelementen 18 des Bodens 10 einerseits und der Hebeauflage 30 bzw. den beiden Hebeauflageelementen 32 andererseits hin und her verschiebbar ist.

In dem in Figur 2 gezeigten Zustand wird die unterste Sterilgutbehälteraufnahme 24 mitsamt den auf dieser gelagerten großen Sterilgutbehältern 22 gerade von dem Aufnahmeraum 16 auf die Hebeauflage 30 geschoben.

In dem in Figur 3 gezeigten Zustand ist die unterste Sterilgutbehälteraufnahme 24 mitsamt den auf dieser gelagerten großen Sterilgutbehältern 22 vollständig auf die Hebeauflage 30 geschoben.

Mittels der Linearführung 28 und dem darin integrierten Antrieb kann die Hebeauflage 30 aus der in den Figuren 1 bis 3 gezeigten Position angehoben werden. Beispielsweise kann die Hebeauflage derart angehoben werden, dass sie in einer zweiten Position derart an die Auflageelemente 18 einer von dem Boden 10 beabstandeten Ebene angeordnet ist, dass die zuvor unterste Sterilgutbehälteraufnahme 24 zwischen den Auflageelementen der von dem Boden 10 beabstandeten Ebene und der Hebeauflage 30 hin und her verschiebbar ist. In den in den Figuren 3 und 4 gezeigten Zuständen ist der Transportwagen 4 bis auf die unterste Ebene voll beladen, so dass für eine Rochade von Sterilgutbehälteraufnahmen 24 zunächst eine Sterilgutbehälteraufnahme 24 entfernt werden müsste.

In dem in Figur 4 gezeigten Zustand ist die Hebeauflage 30 mitsamt der zuvor untersten Sterilgutbehälteraufnahme 24 und den auf dieser gelagerten großen Sterilgutbehältern 22 in eine Position angehoben, in welcher eine Person einen der großen Sterilgutbehälter 22 der Sterilgutbehälteraufnahme 24 in ergonomischer Weise entnehmen kann.

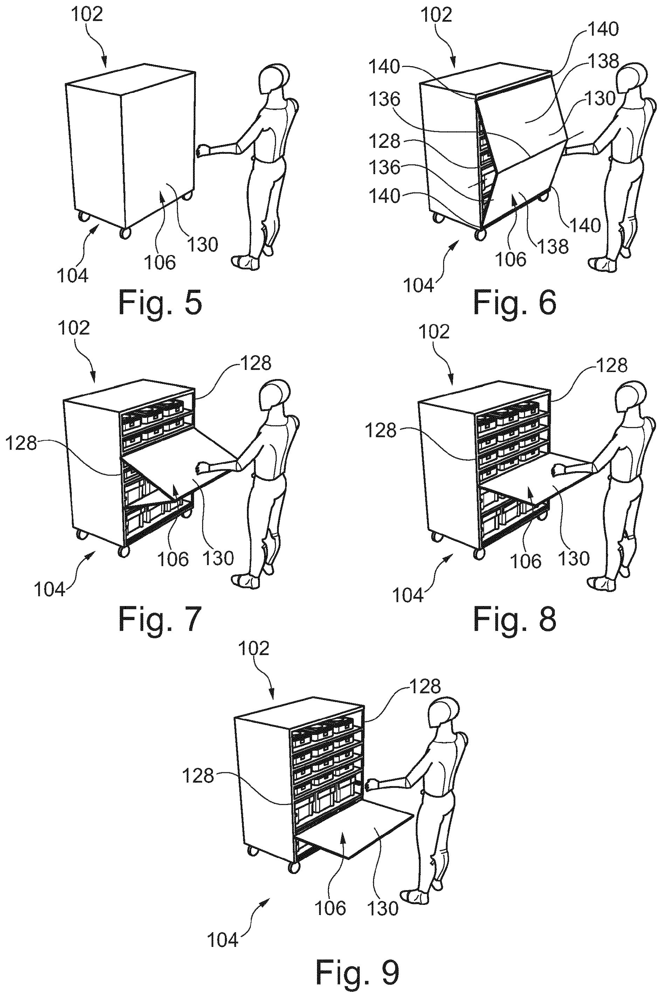

Die Figuren 5 bis 14 zeigen perspektivische Ansichten einer offenbarungsgemäßen Transportvorrichtung 102 gemäß einer ersten Ausführungsform mit einem Transportwagen 104.

Der Transportwagen 104 ist als ein Rollschrank ausgebildet und weist ein Gehäuse 108 mit einem Boden 110, zwei sich von dem Boden 110 nach oben erstreckenden, sich gegenüberliegende Seitenwänden 112 und einer dem Boden 110 gegenüberliegenden Abdeckplatte 114 auf.

Wie in Figur 10 gezeigt, sind der Boden 110, die Seitenwände 112, die Abdeckplatte 114, der Aufnahmeraum 116, Auflageelemente 118, Sterilgutbehälteraufnahmen 124 sowie Rollen 126 des Transportwagens 104 gemäß der ersten Ausführungsform entsprechend dem Boden 10, den Seitenwänden 12, der Abdeckplatte 14, dem Aufnahmeraum 16, den Auflageelementen 18, den Sterilgutbehälteraufnahmen 24 sowie den Rollen 26 des Transportwagens 2 gemäß der nicht von der vorliegenden Erfindung umfassten Ausführungsform ausgebildet.

Im Gegensatz zu der Transportvorrichtung 2 gemäß der nicht von der vorliegenden Erfindung umfassten Ausführungsform weist die Transportvorrichtung 102 gemäß der ersten Ausführungsform eine an dem Transportwagen 104 ausgebildete Hebevorrichtung 106 auf.

Die Hebevorrichtung 106 weist eine als Falttür ausgebildete Hebeauflage 130 auf, die in einem geschlossenen Zustand eine von dem Boden 110, den Seitenwänden 112 und der Abdeckplatte 114 eingerahmte Öffnung abdeckt. Dazu ist an Stirnseiten der Seitenwände 112 jeweils eine senkrecht verlaufende Führungsschiene ausgebildet, die zusammen eine Linearführung 124 für die plattenförmige Hebeauflage 130 bilden.

Wie in Fig. 6 gezeigt, weist die Hebeauflage 130 zwei miteinander um eine Faltachse 136 klappbar verbundene Türblätter 138 auf. Die Hebeauflage 130 ist derart ausgebildet, dass die Faltachse 136 der Türblätter 138 parallel zu dem Boden 110 ausgerichtet ist. Die Türblätter 138 sind jeweils mit einem der Faltachse 136 gegenüberliegenden Ende an entsprechenden Kontaktstellen 140 schwenk- und verschiebbar mit beiden Führungsschienen der Linearführung 128 verbunden. Um gegenüber der Linearführung 128 verschoben zu werden, weisen die Führungsschienen einen (nicht gezeigten) Antrieb bzw. jeweils zwei (nicht gezeigte) Aktoren an den Kontaktstellen 140 auf.

In dem in Figur 5 gezeigten Zustand befinden sich die Türblätter 138 der Hebeauflage 128 in einem auseinandergefalteten Zustand, in welchem die Türblätter 138 senkrecht ausgerichtet sind und die den Aufnahmeraum 116 abdecken.

In dem in Figur 8 gezeigten Zustand befinden sich die Türblätter 138 der Hebeauflage 128 in einem zusammengefalteten Zustand, in welchem die Türblätter 138 waagrecht ausgerichtet sind, so dass auf den Türblättern 138 Sterilgutbehälter 20 bzw. 24 abgestellt werden können bzw. eine der Sterilgutbehälteraufnahmen 124 mit entsprechenden Sterilgutbehältern 20 und/oder 24 auf den Türblättern 138 abgestellt werden kann.

Die Figuren 6 und 7 zeigen Zwischenzustände während eines Zusammenfaltens der Türblätter 138.

Die zusammengefalteten Türblätter 138 können, zum Beispiel ausgehend von dem in Fig. 8 gezeigten Zustand, entlang der Führungsschienen der Linearführung 128 translatorisch abgesenkt und angehoben werden.

In den Figuren 9 und 10 ist gezeigt, wie die zusammengefalteten Türblätter 138 auf eine unterste Ebene verschoben werden, so dass die durch die zusammengefalteten Türblätter 138 gebildete Hebeauflage 130 derart an eine obere Seite des Bodens 110 bzw. an Auflageelemente 118 des Bodens 110 angeordnet sind, dass die entsprechende unterste Sterilgutbehälteraufnahme 124 zwischen der oberen Seite des Bodens 110 bzw. den Auflageelementen 118 des Bodens 110 einerseits und der Hebeauflage 130 andererseits hin und her verschiebbar ist.

Die Figuren 11 bis 13 zeigen den Figuren 2 bis 4 entsprechende Zustände.

In dem in Figur 11 gezeigten Zustand wird die unterste Sterilgutbehälteraufnahme 124 mitsamt den auf dieser gelagerten großen Sterilgutbehältern 22 von dem Aufnahmeraum 16 auf die Hebeauflage 30 geschoben.

In dem in Figur 12 gezeigten Zustand ist die unterste Sterilgutbehälteraufnahme 124 mitsamt den auf dieser gelagerten großen Sterilgutbehältern 22 vollständig auf die Hebeauflage 130 geschoben.

Mittels der Linearführung 128 und dem darin integrierten Antrieb bzw. den darin integrierten Aktoren kann die Hebeauflage 130 aus der in den Figuren 10 bis 12 gezeigten Position angehoben werden. Beispielsweise kann die Hebeauflage 130 derart angehoben werden, dass sie in einer zweiten Position derart an die Auflageelemente 118 einer von dem Boden 110 beabstandeten Ebene angeordnet ist, dass die zuvor unterste Sterilgutbehälteraufnahme 124 zwischen den Auflageelementen der von dem Boden 110 beabstandeten Ebene und der Hebeauflage 130 hin und her verschiebbar ist. In den in den Figuren 12 und 13 gezeigten Zuständen ist der Transportwagen 104 bis auf die unterste Ebene voll beladen, so dass für eine Rochade von Sterilgutbehälteraufnahmen 124 zunächst eine Sterilgutbehälteraufnahme 124 entfernt werden müsste.

In dem in Figur 13 gezeigten Zustand ist die Hebeauflage 30 mitsamt der zuvor untersten Sterilgutbehälteraufnahme 124 und den auf dieser gelagerten großen Sterilgutbehältern 22 in eine Position angehoben, in welcher eine Person einen der großen Sterilgutbehälter 22 der Sterilgutbehälteraufnahme 24 in ergonomischer Weise entnehmen kann.

Figur 14 zeigt einen Zustand, in welchem ein Sterilgutbehälter 22 von der auf der Hebeauflage 130 gelagerten Sterilgutbehälteraufnahme 124 entladen ist.

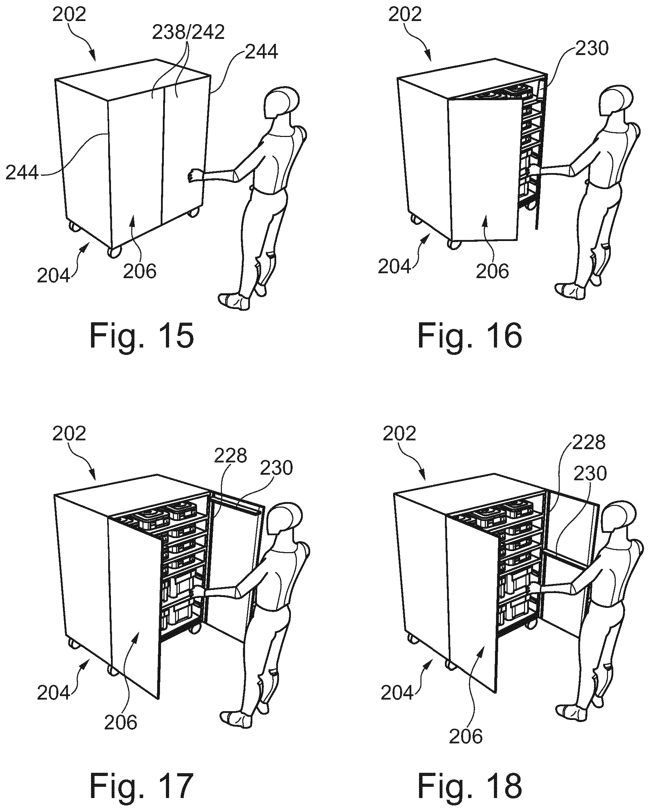

Die Figuren 15 bis 22 zeigen perspektivische Ansichten einer offenbarungsgemäßen Transportvorrichtung 202 gemäß einer zweiten Ausführungsform mit einem Transportwagen 204.

Der Transportwagen 204 ist als ein Rollschrank ausgebildet und weist ein Gehäuse 208 mit einem Boden 210, zwei sich von dem Boden 210 nach oben erstreckenden, sich gegenüberliegende Seitenwänden 212 und einer dem Boden 210 gegenüberliegenden Abdeckplatte 214 auf.

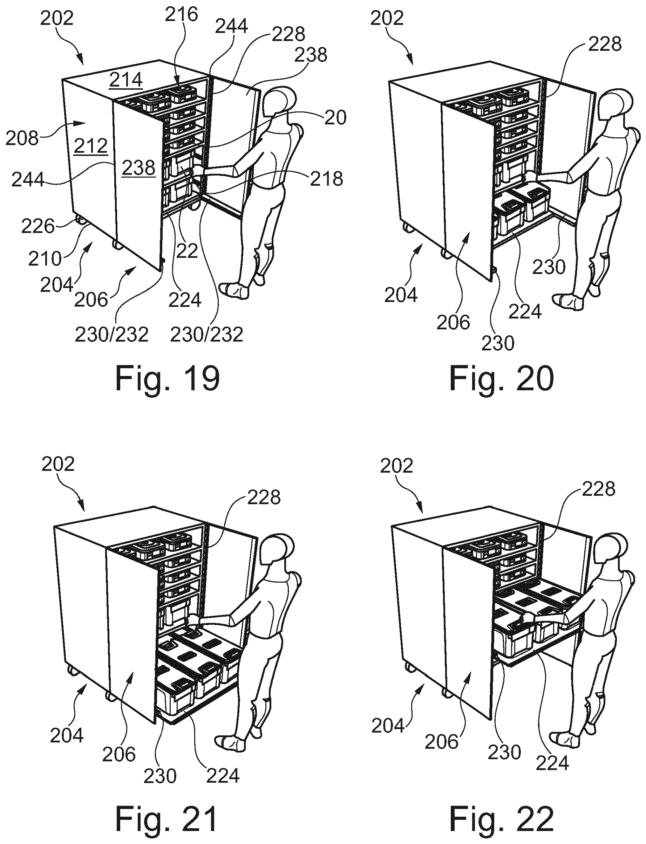

Wie in Figur 19 gezeigt, sind der Boden 210, die Seitenwände 212, die Abdeckplatte 214, der Aufnahmeraum 216, Auflageelemente 218, Sterilgutbehälteraufnahmen 224 sowie Rollen 226 des Transportwagens 204 gemäß der zweiten Ausführungsform entsprechend dem Boden 10, den Seitenwänden 12, der Abdeckplatte 14, dem Aufnahmeraum 16, den Auflageelementen 18, den Sterilgutbehälteraufnahmen 24 sowie den Rollen 26 des Transportwagens 2 gemäß der nicht von der vorliegenden Erfindung umfassten Ausführungsform ausgebildet.

Im Gegensatz zu der Transportvorrichtung 2 gemäß der nicht von der vorliegenden Erfindung umfassten Ausführungsform weist die Transportvorrichtung 202 gemäß der zweiten Ausführungsform wie die Transportvorrichtung 102 gemäß der ersten Ausführungsform eine an dem Transportwagen 204 ausgebildete Hebevorrichtung 206 auf.

Die Hebevorrichtung 206 weist eine doppelflügelige Schwenktür 242 mit zwei Türblättern 238 auf. Die beiden Türblätter 238 sind jeweils an einer Stirnseite der entsprechenden Seitenwand 212 derart über senkrecht verlaufende Scharniere 244 schwenkbar angebracht, dass die Türblätter 238 in einer geschlossenen Stellung (siehe Fig. 15) eine von dem Boden 210, den Seitenwänden 212 und der Abdeckplatte 214 eingerahmte Öffnung abdecken und in einer geöffneten Stellung (siehe zum Beispiel Fig. 17) jeweils die entsprechende Seitenwand 212 verlängern bzw. jeweils in derselben (senkrechten) Ebene ausgerichtet sind wie die direkt benachbarte Seitenwand 212.

Beide Türblätter 238 weisen jeweils ein Hebeauflageelement 232 und zwei als Linearführung 228 dienende Führungsschienen auf. Das jeweilige Hebeauflageelement 232 ist an einer Innenseite des entsprechenden Türblattes 238, die dem Aufnahmeraum 216 zugewandt ist, wenn das Türblatt 238 bzw. die Türblätter 238 in der geschlossenen Stellung sind, parallel zu dem Boden 210 bzw. parallel zur Abdeckplatte 214 bzw. parallel zu den durch die Auflageelemente 218 definierten Ebenen angeordnet. Die jeweiligen Führungsschienen sind an zwei gegenüberliegenden Rändern des entsprechenden Türblattes 238 quer bzw. senkrecht zu dem Boden 210 bzw. zur Abdeckplatte 214 bzw. zu den durch die Auflageelemente 218 definierten Ebenen angeordnet. Die Hebeauflageelemente 232 sind als Leisten ausgebildet, deren beiden Enden schwenk- und verschiebbar mit einer der beiden Führungsschienen eines jeweiligen Türblattes 238 verbunden ist. Die Hebeauflageelemente 232 können entlang der entsprechenden Führungsschienen verschoben werden und relativ zu dem entsprechenden Türblatt 238 in eine ausgeklappte Stellung sowie in eine eingeklappte Stellung verschwenkt bzw. geklappt werden. In der eingeklappten Stellung liegen die Hebeauflageelemente 232 derart an dem entsprechenden Türblatt 238 an, dass die Hebeauflageelemente 232 nicht oder nur teilweise in einen Bereich vor der Aufnahmeraum 216 hineinragen, wenn die Türblätter 238 in der geöffneten Stellung sind. In der ausgeklappten Stellung ragen die Hebeauflageelemente 232 derart von dem entsprechenden Türblatt 238 weg, dass eine Sterilgutbehälteraufnahme 224 auf die beiden Hebeauflageelemente 232 aufgelegt werden kann, wenn die Türblätter 238 in der geöffneten Stellung sind und die beiden Hebeauflageelemente 232 auf einem gemeinsamen Niveau sind.

Zumindest in einer der beiden Führungsschienen eines jeden Türblattes 238, vorzugsweise in beiden Führungsschienen, ist ein Aktor vorgesehen, mittels dessen das entsprechende Hebeauflageelement zumindest verschoben, vorzugsweise verschoben und verschwenkt, werden kann. Die beiden Hebeauflageelemente 232 bilden zusammen die Hebeauflage 230.

In dem in Fig. 15 gezeigten Zustand sind die Türblätter 238 in der geschlossenen Stellung.

In dem in Fig. 17 gezeigten Zustand sind die Türblätter 238 in der geöffneten Stellung und die Hebeauflageelemente 238 bzw. Hebeauflage 230 auf Höhe einer unteren Seite der Abdeckplatte 214.

Fig. 16 zeigt einen Zustand während des Öffnens der Schwenktür 242 bzw. der Türblätter 238.

In den Figuren 18 und 19 ist gezeigt, wie die Hebeauflage 230 auf eine unterste Ebene verschoben wird, so dass die durch Hebeauflageelemente 232 gebildete Hebeauflage 230 derart an einer oberen Seite des Bodens 210 bzw. an Auflageelemente 218 des Bodens 210 angeordnet ist, dass die entsprechende unterste Sterilgutbehälteraufnahme 224 zwischen der oberen Seite des Bodens 210 bzw. den Auflageelementen 218 des Bodens 210 einerseits und der Hebeauflage 230 andererseits hin und her verschiebbar ist.

Die Figuren 20 bis 22 zeigen den Figuren 2 bis 4 entsprechende Zustände.

In dem in Figur 20 gezeigten Zustand wird die unterste Sterilgutbehälteraufnahme 224 mitsamt den auf dieser gelagerten großen Sterilgutbehältern 22 von dem Aufnahmeraum 216 auf die Hebeauflage 230 geschoben.

In dem in Figur 21 gezeigten Zustand ist die unterste Sterilgutbehälteraufnahme 224 mitsamt den auf dieser gelagerten großen Sterilgutbehältern 22 vollständig auf die Hebeauflage 230 geschoben.

Mittels der Linearführung 228 und dem darin integrierten Antrieb bzw. den darin integrierten Aktoren kann die Hebeauflage 230 aus der in den Figuren 19 bis 21 gezeigten Position angehoben werden. Beispielsweise kann die Hebeauflage 230 derart angehoben werden, dass sie in einer zweiten Position derart an die Auflageelemente 218 einer von dem Boden 210 beabstandeten Ebene angeordnet ist, dass die zuvor unterste Sterilgutbehälteraufnahme 224 zwischen den Auflageelementen der von dem Boden 210 beabstandeten Ebene und der Hebeauflage 230 hin und her verschiebbar ist. In den in den Figuren 21 und 22 gezeigten Zuständen ist der Transportwagen 204 bis auf die unterste Ebene voll beladen, so dass für eine Rochade von Sterilgutbehälteraufnahmen 224 zunächst eine Sterilgutbehälteraufnahme 224 entfernt werden müsste.

In dem in Figur 22 gezeigten Zustand ist die Hebeauflage 230 mitsamt der zuvor untersten Sterilgutbehälteraufnahme 224 und den auf dieser gelagerten großen Sterilgutbehältern 22 in eine Position angehoben, in welcher eine Person einen der großen Sterilgutbehälter 22 der Sterilgutbehälteraufnahme 24 in ergonomischer Weise entnehmen kann.

Die offenbarungsgemäßen Hebeauflagen 30; 130 bzw. 230 bzw. die entsprechenden Antriebe können insbesondre mittels (nicht gezeigter) Bedienelemente gesteuert werden. Insbesondere sind verschiedene Positionen der Hebeauflagen in den Bedienelementen bzw. in entsprechenden Steuergeräten gespeichert, welche den durch die Auflageelementen 18; 118 bzw. 218 definierten Ebenen entsprechen. Vorzugsweise sind die Bedienelemente für die Hebeauflagen 103 und 230 an einem der Türblätter 138 bzw. 238 angeordnet. Die Sterilgutbehälteraufnahmen 124 bzw. 224 sind insbesondere derart ausgebildet, dass diese formschlüssig mit der entsprechenden Linearführung 128 bzw. 228 zusammenwirken kann, so dass ein Lösen der Sterilgutbehälteraufnahmen 124 bzw. 224 von der Transportvorrichtung 102 bzw. 202 nicht oder zumindest nicht ohne weiteres Zutun möglich ist.

Die offenbarungsgemäßen Transportwägen 4; 104 bzw. 204 können insbesondere derart ausgebildet sein, dass die Sterilgutbehälteraufnahmen 24; 124 bzw. 224 mittels dafür vorgesehener Antriebe entlang der Auflageelemente 18; 118 bzw. 218 aus dem und/oder in den Aufnahmeraum 16; 116 bzw. 216 geschoben werden können.

Die offenbarungsgemäßen Transportwägen 4; 104 bzw. 204 können als fahrerlose Transportsysteme (FTS) bzw. Automated Guided Vehicles (AGV) ausgebildet sein.

Die offenbarungsgemäßen Transportwägen 104 bzw. 204 können jeweils mit einem mechatronischen Schloss versehen sein, mittels dessen die jeweilige Tür in der geschlossenen Stellung verriegelbar ist. Das jeweilige Schloss kann vorzugsweise derart ausgebildet sein, dass eine Freischaltung des Schlosses erfolgt, wenn der entsprechende Transportwagen 104 bzw. 204 an vorbestimmten Positionen bzw. einer vorbestimmten Position ist.

### Bezugszeichenliste

- 2; 102; 202: Transportvorrichtung
- 4; 104, 204: Transportwagen
- 6; 106; 206: Hebevorrichtung
- 8; 108; 208: Gehäuse
- 10; 110; 210: Boden
- 12; 112; 212: Seitenwand
- 14; 114; 214: Abdeckplatte
- 16; 116; 216: Aufnahmeraum
- 18; 118; 218: Auflageelement
- 20, 22: Sterilgutbehälter
- 24; 124; 224: Sterilgutbehälteraufnahme
- 26; 126; 226: Rolle
- 28; 128, 228: Linearführung
- 30; 130; 230: Hebeauflage
- 32; 232: Hebeauflageelement
- 34: Verbindungsteg
- 136: Faltachse
- 138; 238: Türblatt
- 140: Kontaktstelle
- 242: Schwenktür
- 244: Scharnier

## Patentansprüche

1. Transportvorrichtung (102; 202) zum Transport zumindest eines Sterilgutbehälters (20; 22) mit
einem Aufnahmeraum (116; 216), der in einem beweglichen Transportwagen (104; 204) ausgebildet ist und der auf einer ersten Ebene und auf einer zu der ersten Ebene parallelen zweiten Ebene Auflageelemente (118; 218) aufweist, auf denen eine Sterilgutbehälteraufnahme (124; 224) lagerbar ist, und
einer Hebeauflage (130; 230), die in einer ersten Position derart an die Auflageelemente der ersten Ebene anordenbar ist, dass die Sterilgutbehälteraufnahme (124; 224) zwischen den Auflageelementen (118; 218) der ersten Ebene und der Hebeauflage (130; 230) hin und her verschiebbar ist, wobei
die Hebeauflage (130; 230) ausgebildet ist, in einer zweiten Position derart an die Auflageelemente (118; 218) der zweiten Ebene angeordnet werden zu können, dass die Sterilgutbehälteraufnahme (124; 224) zwischen den Auflageelementen (118; 218) der zweiten Ebene und der Hebeauflage (130; 230) hin und her verschiebbar ist,
die Transportvorrichtung (102; 202) eine Linearführung (128; 228) aufweist, die quer zu der ersten und zweiten Ebene verläuft und mittels derer die Hebeauflage (130; 230) von deren ersten Position in deren zweite Position geführt werden kann, und
die Transportvorrichtung (102; 202) einen Antrieb aufweist, mittels dessen die Hebeauflage (130; 230) entlang der Linearführung (128; 228) von der ersten Position in die zweite Position bewegbar ist,
**dadurch gekennzeichnet, dass**
die Linearführung (128; 228), der Antrieb und die Hebeauflage (130; 230) an dem Transportwagen (104; 204) ausgebildet sind.

2. Transportvorrichtung (202) nach Anspruch 1, **dadurch gekennzeichnet, dass**
der Transportwagen (204) einen Rahmen aufweist, der eine Öffnung einfasst, durch welche der Sterilgutbehälter (20; 22) in den oder aus dem Aufnahmeraum (216) bewegt werden kann,
der Transportwagen (204) eine Tür (242) aufweist, die schwenkbar an einer äußeren Stirnseite des Rahmens angebracht ist, und
die Linearführung (228), der Antrieb sowie die Hebeauflage (230) an einer Innenseite der Tür (242) derart ausgebildet sind, dass die Hebeauflage (230) in einer geöffneten Stellung der Tür (242) parallel zu den Auflageelementen (218) des Aufnahmeraums (216) ausgerichtet sein kann.

3. Transportvorrichtung (202) nach Anspruch 2, **dadurch gekennzeichnet, dass** die Hebeauflage (230) derart schwenkbar mit der Linearführung (228) verbunden ist, dass die Hebeauflage (230) aus einer ausgeklappten Stellung, in welcher die Hebeauflage (230) in der geöffneten Stellung der Tür (242) parallel zu den Auflageelementen (218) des Aufnahmeraums (216) ausgerichtet ist, in eine eingeklappte Stellung, in welcher die Hebeauflage (230) parallel zu der Tür (242) ausgerichtet ist, geklappt werden kann.

4. Transportvorrichtung (202) nach Anspruch 2 oder 3, **dadurch gekennzeichnet, dass**
die Tür (242) als Stulptür mit einem ersten Türblatt (238) und einem zweiten Türblatt (238) ausgebildet ist, und
die Hebeauflage (230) ein erstes Hebeauflageelement (232) aufweist, dass an dem ersten Türblatt (238) ausgebildet ist, sowie ein zweites Hebeauflagenelement (232) aufweist, das an dem zweiten Türblatt (238) ausgebildet ist.

5. Transportvorrichtung (102) nach Anspruch 1, **dadurch gekennzeichnet, dass** der Transportwagen (104) einen Rahmen aufweist, der eine Öffnung einfasst, durch welche der Sterilgutbehälter (20; 22) in den oder aus dem Aufnahmeraum (116) bewegt werden kann, und die Linearführung (128), der Antrieb sowie die Hebeauflage (130) an einer äußeren Stirnseite des Rahmens ausgebildet sind.

6. Transportvorrichtung (102) nach Anspruch 5, **dadurch gekennzeichnet, dass** die Hebeauflage (130) als eine Tür ausgebildet ist, die derart schwenk- und verschiebbar mit der Linearführung (128) verbunden ist, dass sie in einer Abdeckungsorientierung die von dem Rahmen eingefasste Öffnung zumindest größtenteils abdecken kann und in einer quer zur Abdeckungsorientierung ausgerichteten Hebeorientierung parallel zu den Auflageelementen (118) des Aufnahmeraums (116) ausgerichtet ist.

7. Transportvorrichtung (102) nach Anspruch 6, **dadurch gekennzeichnet, dass** die Tür als eine Falttür ausgebildet ist, deren Türblätter (138) in einem auseinandergefalteten Zustand in der Abdeckungsorientierung und in einem zusammengefalteten Zustand in der Hebeorientierung ausgerichtet sind.

8. Transportvorrichtung (102) nach Anspruch 6, **dadurch gekennzeichnet, dass** der Antrieb je Türblatt (138) zumindest einen Aktor zum Verschieben des jeweiligen Türblatts (138) entlang der Linearführung (128) aufweist.

9. Transportvorrichtung (102; 202) nach Anspruch 2, 3, 4, 7 oder 8, **dadurch gekennzeichnet, dass** an der Tür zumindest ein relativ zur Tür bewegliches Abstützelement vorgesehen ist, das in eine Abstützposition bewegbar ist, in welcher das Abstützelement die Tür in deren geöffneten Stellung der Tür gegenüber einem planen Untergrund, auf dem der Transportwagen (104; 204) lagert, abstützen kann.

10. Verwendung einer Transportvorrichtung (102; 202) nach einem der Ansprüche 1 bis 9 zum Transport zumindest eines Sterilgutbehälters (20; 22).

## Claims

1. A transport device (102; 202) for transporting at least one sterile-product container (20; 22) having
a receiving space (116; 216) which is configured in a movable transport trolley (104; 204) and which has support elements (118; 218) on a first plane and on a second plane parallel to the first plane, wherein a sterile-product container receptacle (124; 224) is supportable on the support elements (118; 218), and
a lifting support (130; 230) which is arrangeable in a first position on the support elements of the first plane in such a way that the sterile-product container receptacle (124; 224) is displaceable back and forth between the support elements (118; 218) of the first plane and the lifting support (130; 230), wherein
the lifting support (130; 230) is configured to be arrangeable in a second position on the support elements (118; 218) of the second plane in such a way that the sterile-product container receptacle (124; 224) is displaceable back and forth between the support elements (118; 218) of the second plane and the lifting support (130; 230),
the transport device (102; 202) has a linear guide (128; 228) which extends transversely to the first and second planes and via which the lifting support (130; 230) can be guided from its first position to its second position, and
the transport device (102; 202) has a drive via which the lifting support (130; 230) is movable along the linear guide (128; 228) from the first position to the second position,
**characterized in that**
the linear guide (128; 228), the drive, and the lifting support (130; 230) are configured on the transport trolley (104; 204).

2. The transport device (202) according to claim 1, **characterized in that**
the transport trolley (204) has a frame surrounding an opening through which the sterile-product container (20; 22) can be moved into or out of the receiving space (216),
the transport trolley (204) has a door (242) pivotably mounted on an outer front side of the frame, and
the linear guide (228), the drive as well as the lifting support (230) are configured on an inner side of the door (242) in such a way that the lifting support (230) can be aligned parallel to the support elements (218) of the receiving space (216) in an open position of the door (242).

3. The transport device (202) according to claim 2, **characterized in that** the lifting support (230) is pivotably connected to the linear guide (228) in such a way that the lifting support (230) can be folded from a folded-out position, in which the lifting support (230) is aligned parallel to the support elements (218) of the receiving space (216) in the open position of the door (242), into a folded-in position, in which the lifting support (230) is aligned parallel to the door (242).

4. The transport device (202) according to claim 2 or 3, **characterized in that**
the door (242) is configured as a French door having a first door leaf (238) and a second door leaf (238), and
the lifting support (230) comprises a first lifting-support element (232) configured on the first door leaf (238) and a second lifting-support element (232) configured on the second door leaf (238).

5. The transport device (102) according to claim 1, **characterized in that** the transport trolley (104) has a frame surrounding an opening through which the sterile-product container (20; 22) can be moved into or out of the receiving space (116), and the linear guide (128), the drive, and the lifting support (130) are configured on an outer front side of the frame.

6. The transport device (102) according to claim 5, **characterized in that** the lifting support (130) is configured as a door which is pivotably and displaceably connected to the linear guide (128) in such a way that the door can at least largely cover the opening surrounded by the frame in a covering orientation, and the door is aligned parallel to the support elements (118) of the receiving space (116) in a lifting orientation aligned transversely to the covering orientation.

7. The transport device (102) according to claim 6, **characterized in that** the door is configured as a folding door whose door leaves (138) are aligned in the covering orientation in an unfolded state and in the lifting orientation in a folded state.

8. The transport device (102) according to claim 6, **characterized in that** the drive per door leaf (138) has at least one actuator for displacing the respective door leaf (138) along the linear guide (128).

9. The transport device (102; 202) according to claim 2, 3, 4, 7 or 8, **characterized in that** at least one bracing element movable relative to the door is provided on the door, wherein the bracing element is movable into a bracing position in which the bracing element can support the door in its open position relative to a flat base on which the transport trolley (104; 204) is supported.

10. Use of a transport device (102; 202) according to one of claims 1 to 9 for transporting at least one sterile-product container (20; 22).

## Revendications

1. Dispositif de transport (102 ; 202) pour le transport au moins d'un récipient de produit stérile (20 ; 22) avec
un espace de réception (116 ; 216) qui est formé dans un chariot de transport (104 ; 204) mobile et qui présente des éléments de support (118 ; 218) sur un premier plan et sur un second plan parallèle au premier plan, éléments sur lesquels un logement de récipient de produit stérile (124 ; 224) peut être entreposé, et
un support de levage (130 ; 230) qui peut être agencé dans une première position au niveau des éléments de support du premier plan de telle manière que le logement de récipient de produit stérile (124 ; 224) soit alternativement coulissant entre les éléments de support (118 ; 218) du premier plan et le support de levage (130 ; 230), dans lequel
le support de levage (130 ; 230) est formé afin de pouvoir être agencé dans une seconde position au niveau des éléments de support (118 ; 218) du second plan de telle manière que le logement de récipient de produit stérile (124 ; 224) soit alternativement coulissant entre les éléments de support (118 ; 218) du second plan et le support de levage (130 ; 230),
le dispositif de transport (102 ; 202) présente un guidage linéaire (128 ; 228) qui s'étend transversalement au premier et au second plan et au moyen duquel le support de levage (130 ; 230) peut être guidé de sa première position dans sa seconde position, et
le dispositif de transport (102 ; 202) présente un entraînement au moyen duquel le support de levage (130 ; 230) peut être déplacé de la première position dans la seconde position le long du guidage linéaire (128 ; 228),
**caractérisé en ce que**
le guidage linéaire (128 ; 228), l'entraînement et le support de levage (130 ; 230) sont formés au niveau du chariot de transport (104 ; 204).

2. Dispositif de transport (202) selon la revendication 1, **caractérisé en ce que**
le chariot de transport (204) présente un cadre qui borde une ouverture par laquelle le récipient de produit stérile (20 ; 22) peut être déplacé dans ou hors de l'espace de réception (216),
le chariot de transport (204) présente une porte (242) qui est montée de manière pivotante au niveau d'un côté avant extérieur du cadre et
le guidage linéaire (228), l'entraînement ainsi que le support de levage (230) sont formés au niveau d'un côté intérieur de la porte (242) de telle manière que le support de levage (230) puisse être orienté parallèlement aux éléments de support (218) de l'espace de réception (216) dans une position ouverte de la porte (242).

3. Dispositif de transport (202) selon la revendication 2, **caractérisé en ce que** le support de levage (230) est relié de manière pivotante au guidage linéaire (228) de telle manière que le support de levage (230) puisse être rabattu depuis une position dépliée dans laquelle le support de levage (230) est orienté parallèlement aux éléments de support (218) de l'espace de réception (216) dans la position ouverte de la porte (242) dans une position repliée dans laquelle le support de levage (230) est orienté parallèlement à la porte (242).

4. Dispositif de transport (202) selon la revendication 2 ou 3, **caractérisé en ce que**
la porte (242) est formée comme porte à recouvrement avec un premier vantail de porte (238) et un second vantail de porte (238) et
le support de levage (230) présente un premier élément de support de levage (232) qui est formé au niveau du premier vantail de porte (238) et présente un second élément de support de levage (232) qui est formé au niveau du second vantail de porte (238).

5. Dispositif de transport (102) selon la revendication 1, **caractérisé en ce que** le chariot de transport (104) présente un cadre qui borde une ouverture par laquelle le récipient de produit stérile (20 ; 22) peut être déplacé dans ou hors de l'espace de réception (116), et le guidage linéaire (128), l'entraînement ainsi que le support de levage (130) sont formés au niveau d'un côté avant extérieur du cadre.

6. Dispositif de transport (102) selon la revendication 5, **caractérisé en ce que** le support de levage (130) est formé comme une porte qui est reliée de manière pivotante et coulissante mobile au guidage linéaire (128) de telle manière qu'il puisse recouvrir au moins en grande partie l'ouverture bordée par le cadre dans une orientation de recouvrement et soit orienté dans une orientation de levage orientée transversalement à l'orientation de recouvrement parallèlement aux éléments de support (118) de l'espace de réception (116).

7. Dispositif de transport (102) selon la revendication 6, **caractérisé en ce que** la porte est formée comme porte pliante, dont les vantaux de porte (138) sont orientés dans un état déplié dans l'orientation de recouvrement et dans un état replié dans l'orientation de levage.

8. Dispositif de transport (102) selon la revendication 6, **caractérisé en ce que** l'entraînement présente par vantail de porte (138) au moins un actionneur pour déplacer le vantail de porte (138) respectif le long du guidage linéaire (128).

9. Dispositif de transport (102 ; 202) selon la revendication 2, 3, 4, 7 ou 8, **caractérisé en ce qu'**au moins un élément d'appui mobile par rapport à la porte est prévu au niveau de la porte, élément qui peut être déplacé dans une position d'appui dans laquelle l'élément d'appui peut supporter la porte dans sa position ouverte de porte par rapport à un sol plan sur lequel le chariot de transport (104 ; 204) est entreposé.

10. Utilisation d'un dispositif de transport (102 ; 202) selon l'une quelconque des revendications 1 à 9 pour le transport au moins d'un récipient de produit stérile (20 ; 22).
